# EUROPÄISCHE PATENTSCHRIFT

(11) **EP 3 213 745 B1**
(45) Veröffentlichungstag und Bekanntmachung des Hinweises auf die Patenterteilung: **05.05.2021**
(21) Anmeldenummer: 17158719.9
(22) Anmeldetag: 01.03.2017
(51) Int. Cl.: A61K 9/00, A61K 47/10, A61K 47/14, A61K 31/192

(54) **GALENISCHE ZUBEREITUNG VON NSAID**
GALENICAL FORMULATION OF NSAIDS
PREPARATION GALENIQUE DE NSAID

(30) Priorität: 01.03.2016 EP 16158111
(43) Veröffentlichungstag der Anmeldung: 06.09.2017
(73) Patentinhaber: CHEMISCHE FABRIK KREUSSLER & CO. GMBH, 65203 Wiesbaden (DE)
(72) Erfinder: Heydt-Lotter, Silke, 65201 Wiesbaden (DE); Lotter, Matthias, 65201 Wiesbaden (DE); Travers, Stephan, 65187 Wiesbaden (DE)
(74) Vertreter: dompatent von Kreisler Selting Werner - Partnerschaft von Patent- und Rechtsanwälten mbB

(56) Entgegenhaltungen:
- EP-A1- 0 672 422
- EP-A1- 1 716 845
- EP-A1- 1 964 552
- EP-A1- 2 620 146
- EP-A2- 0 215 423
- WO-A1-2011/063531

## Beschreibung

Die vorliegende Anmeldung betrifft eine galenische Zubereitung von nichtsteroidalen Antiphlogistika in Form eines Gels.

Nichtsteroidale Antirheumatika, welche auch als nichtsteroidale Antiphlogistika bezeichnet werden, sind Schmerzmittel. Sie hemmen Symptome von Entzündungsprozessen wie Schmerz und Schwellung. Die am häufigsten zu beobachtenden Nebenwirkungen bei oraler Einnahme sind Magenschleimhautschädigungen. Aus diesem Grund begegnet die orale Verabreichung nichtsteroidaler Antiphlogistika (NSAID) zunehmend Bedenken des Verbrauchers.

Neben der oralen Aufnahme besteht auch die Möglichkeit einer transdermalen Aufnahme. Die transdermale Aufnahme von NSAID in Form von Cremes, Gelen oder Sprays haben gegenüber der systemischen Schmerzbehandlung die Vorteile, dass die Konzentration im Blutplasma deutlich geringer ist, was zu einer besseren Verträglichkeit führt. Zudem wird der manuellen Behandlung (dem Einmassieren des Präparates) ein positiver psychologischer und schmerzreduzierender Effekt zugeschrieben. Weiterhin ist die transdermale Therapie kostengünstig.

Es hat sich jedoch gezeigt, dass insbesondere bei Gelen das Auftragen auf und Einmassieren in die Haut vom Verbraucher als unangenehm empfunden wird.

Dies ist insbesondere darauf zurückzuführen, dass bei der manuellen Behandlung, also während des Einreibens, die Gele verklumpen, so dass sich Kügelchen auf der Haut ausbilden. Der durch ein Gel weiterhin hervorgerufene kühlende Effekt wird hierdurch vermindert. Insbesondere ist die Haptik hierdurch verschlechtert, was zu einer geringeren Compliance führen kann. Zumindest wird die manuelle Behandlung durch Einreiben/Einmassieren reduziert, was zu einer Reduktion der hierdurch erreichten positiven Wirkung führt. Es besteht somit Bedarf an einer galenischen Zubereitung von NSAID, die auch beim Auftragen und Einreiben eine angenehme Haptik aufweist.

Überraschenderweise hat sich gezeigt, dass eine wässrige Zusammensetzung in Form eines Gels gemäß Anspruch 1 die Nachteile aus dem Stand der Technik vermeidet. Bevorzugte Ausführungsformen sind in den abhängigen Ansprüchen offenbart. Insbesondere die Ausbildung von Gel-Kügelchen beim Einreiben des Gels auf der Haut bleibt überraschenderweise aus.

Die der vorliegenden Erfindung zu Grunde liegende Aufgabe wird somit gelöst durch eine wässrige Zusammensetzung in Form eines Gels für die transdermale Aufnahme wenigstens eines nicht-steroidalen Antiphlogistikums (NSAID) bestehend aus:
a. wenigstens einem nicht-steroidales Antiphlogistikum in einem Anteil von 1 Gew.-% bis 10 Gew.-%,
b. wenigstens einem Verdickungsmittel, welches in der Lage ist, ein dreidimensionales Netzwerk eines Gels auszubilden, in einem Anteil von 3 Gew.-% bis 40 Gew.-%,
c. Dimethylisosorbit als ersten Lösungsvermittler zur Verbesserung der Löslichkeit des wenigstens einen NSAID in dem aliphatischen Alkohol in einem Anteil von 2 Gew.-% bis 35 Gew.-%,
d. Isopropylmyristat als zweiten Lösungsvermittler zur Verbesserung der Verreibbarkeit der Zusammensetzung auf der Haut, in einem Anteil von 0,5 Gew.-% bis 10 Gew.-%,
e. wenigstens einen aliphatischen Alkohol der allgemeinen Formel CₙH₂ₙ₊₁OH, wobei n eine ganze natürliche Zahl von 2 bis 10 ist, einem Anteil von 10 Gew.-% oder mehr und
f. Wasser in einem Anteil von 40 Gew.-% oder mehr,
wobei die Gesamtmenge an Lösungsvermittler 3 Gew.-% bis 40 Gew.-% beträgt und das Gewichtsverhältnis von erstem Lösungsvermittler zu zweitem Lösungsvermittler 10:1 bis 2:1 beträgt, und
das Gesamtgewicht der Zusammensetzung 100 Gew.-% beträgt.

Das erfindungsgemäße Gel, also die erfindungsgemäße Zusammensetzung, weist Wasser in einem Anteil von 40 Gew.-% oder mehr auf. Wasser ist damit das wesentliche Lösungsmittel, so dass es sich bei dem Gel um eine wässrige Zusammensetzung handelt. Die erfindungsgemäße Zusammensetzung kann weitere Lösungsmittel, wie beispielsweise Alkohol, aufweisen. Der Anteil an weiteren Lösungsmitteln ist jedoch immer geringer als der Anteil an Wasser.

Ein Gel ist ein System, das aus mindestens zwei Komponenten besteht. Die feste Komponente, der Strukturbildner oder das Verdickungsmittel, bildet ein dreidimensionales Netzwerk, dessen Poren durch eine oder mehrere Flüssigkeiten ausgefüllt werden. In der flüssigen Komponente (Flüssigkeit) können weitere Bestandteile gelöst, suspendiert oder dispergiert enthalten sein. Bevorzugt sind die weiteren Bestandteile in der Flüssigkeit gelöst. Die flüssige Komponente ist dadurch in der festen immobilisiert. Beide Komponenten durchdringen sich vollständig. Bei Raumtemperatur behält das Gel ohne äußere Einwirkung im Wesentlichen seine vorgegebene Form. Es kann jedoch ohne größeren Kraftaufwand auf einer Oberfläche, beispielsweise auf der Haut eines Menschen, eingerieben werden.

Gel ist dabei eine von Gelatine abgeleitete Bezeichnung aus der Kolloidchemie für formbeständige, leicht deformierbare, an Flüssigkeiten reiche Systeme aus mindestens zwei Komponenten (siehe beispielsweise Römpp Chemie Lexikon, Cm-G, Band 2, 1990, Georg Thieme Verlag). Eine Komponente ist ein fester, kolloid zerteilter Stoff mit langen oder stark verzweigten Teilchen, welche oft als Strukturbildner, Verdickungsmittel oder Geliermittel bezeichnet wird. Strukturbildner, Verdickungsmittel und Geliermittel werden in der vorliegenden Anmeldung synonym verwendet. Eine zweite Komponente ist eine Flüssigkeit, welche als Dispersionsmittel für den Verdicker dient. Dabei ist die feste Substanz kohärent, dass heißt sie bildet im Dispersionsmittel ein räumliches Netzwerk, wobei die Teilchen durch Neben- oder Hauptvalenzen an verschiedenen Punkten (Haftpunkte) aneinanderhaften.

Die erfindungsgemäße Zusammensetzung in Form eines Gels weist somit wenigstens einen Strukturbildner und wenigstens eine Flüssigkeit auf. Der Strukturbildner ist für die Ausbildung des dreidimensionalen Netzwerkes des Gels verantwortlich. Durch den Strukturbildner behält das Gel ohne äußere Einwirkung seine Form. Der Strukturbildner ist somit ein für die Galenik der erfindungsgemäßen Zusammensetzung verantwortliches Polymer, welches in der Lage ist, ein dreidimensionales Netzwerk auszubilden.

Ein Anteil von 3 Gew.-% bis 40 Gew.-% an Strukturbildner ist dabei einerseits ausreichend und gleichzeitig notwendig, um die weiteren Komponenten in Lösung zu einem Gel zusammenzuhalten. Als Strukturbildner können an sich bekannte Polymere eingesetzt werden, wie beispielsweise Gelatine, Bentonite, Polysaccharide, Pektine und andere. Geeignete Strukturbildner sind dem Fachmann hinlänglich bekannt. Bevorzugt ist der Strukturbildner ein amphiphiles Blockcopolymer aus Ethylenoxid- und Propylenoxideinheiten. Es hat sich gezeigt, dass entsprechende Blockcopolymere besonders stabile Netzwerke ausbilden, wobei jedoch gleichzeitig ein Gel erhalten wird, das gut auf die Haut aufzutragen und dort zu verteilen ist, ohne dass sich die Struktur des Gels ändert.

Bevorzugt wird ein Poloxamer als Strukturbildner eingesetzt. Poloxamere sind tensidartige Blockcopolymere mit einem zentralen Polypropylenglykolteil, der an beiden Kettenenden mit jeweils einem Makrogolanteil verknüpft ist. Besonders bevorzugt ist der Strukturbildner Poloxamer 407.

Die dreistellige Ziffernfolge definiert dabei den Massenanteil der Ethylenoxideinheiten. Die letzte Ziffer mit dem Faktor 10 multipliziert gibt in etwa den relativen Massenanteil der Ethylenoxideinheiten in Prozent an. Die voranstehenden Ziffern codieren die relative Molekülmasse des Polypropylenglykol-Blockes.

Bevorzugt weist die Zusammensetzung den wenigstens einen Strukturbildner in einem Anteil von 5 Gew.-% bis 35 Gew.-%, insbesondere von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% oder von 8 Gew.-% bis 12 Gew.-%, jeweils bezogen auf das Gesamtgewicht der Zusammensetzung von 100 Gew.-%, auf.

Weiterhin für die Galenik verantwortlich ist die Flüssigkeit, welche zusammen mit dem Strukturbildner das Gel bildet. Die erfindungsgemäße Zusammensetzung weist zum einen Wasser als Flüssigkeit auf. Darüber hinaus umfasst sie wenigstens einen aliphatischen Alkohol. Der Anteil an Wasser beträgt 40 Gew.-% oder mehr. Der Anteil des wenigstens einen aliphatischen Alkohols beträgt 10 Gew.-% oder mehr. Wasser und Alkohol stellen Lösungsmittel für das NSAID dar.

Das erfindungsgemäße Gel weist weiterhin wenigstens ein nichtsteroidales Antiphlogistikum auf. Dieses ist vorzugsweise ausgewählt aus Arylpropionsäure und deren Derivaten, Arylessigsäure und deren Derivaten, Indolessigsäure und deren Derivaten, sowie Oxicamen. Dabei kann das erfindungsgemäße Gel auch Mischungen der genannten Komponenten aufweisen. Insbesondere weist das Gel ein oder mehrere NSAID, ausgewählt aus Ibuprofen, Flurbiprofen, Naproxen, Ketoprofen und Tiaprofensäure auf. Diese können gut über die Haut aufgenommen werden, so dass ein für eine Wirkung ausreichender Wirkstoffgehalt erreicht werden kann. Besonders bevorzugt handelt es sich bei der wässrigen Zusammensetzung in Form eines Gels gemäß der vorliegenden Erfindung um ein solches für die transdermale Aufnahme von Ibuprofen, wobei Ibuprofen als einziges NSAID in der Zusammensetzung enthalten ist.

Die erfindungsgemäße Zusammensetzung umfasst das wenigstens eine NSAID in einer solchen Konzentration, dass eine schmerzlindernde Wirkung erreicht wird. Hohe Konzentrationen bewirken entsprechend höhere Wirkstoffspiegel im Zielgewebe. Dabei ist auch die von Patienten üblicherweise aufgetragene Menge zu berücksichtigen. Es hat sich gezeigt, dass ein Anteil von 1 Gew.-% bis 10 Gew.-% an NSAID üblicherweise ausreichend ist, um einen Wirkstoffspiegel im Zielgewebe, der eine schmerzlindernde Wirkung ermöglicht, zu erreichen.

Bevorzugt beträgt der Anteil 2 Gew.-% bis 8 Gew.-%, insbesondere 3 Gew.-% bis 7 Gew.-%, besonderes bevorzugt 5 Gew.-%, jeweils bezogen auf das Gesamtgewicht der Zusammensetzung von 100 Gew.-%. Gerade ein Anteil von 5 Gew.-% an NSAID ist der Kompromiss zwischen der Bereitstellung eines kostengünstigen Produktes einerseits und einem ausreichenden Wirkstoffspiegel andererseits, so dass dieser Wert und insbesondere der Bereich von 3 Gew.-% bis 7 Gew.-% für das NSAID bevorzugt ist.

Die erfindungsgemäße Zusammensetzung (das Gel) weist weiterhin wenigstens zwei voneinander verschiedene Lösungsvermittler in einem Anteil von 3 Gew.-% bis 40 Gew.-% auf. Es ist erfindungsgemäß auch möglich, dass die Zusammensetzung 3 oder mehr voneinander verschiedene Lösungsvermittler aufweist.

Überraschenderweise hat sich gezeigt, dass die Kombination aus Lösungsvermittler und Strukturbildner in den genannten Mengenbereichen dafür sorgt, dass eine stabile Gelstruktur erhalten wird, die beim Auftragen auf ein Einreiben in die Haut eine angenehme Haptik aufweist. Eine Bildung krümeliger Strukturen, wie im Stand der Technik sonst üblich, erfolgt hier gerade nicht.

Die Zusammensetzung weist erfindungsgemäß 2 Lösungsvermittler auf, wobei einer dafür sorgt, dass das NSAID in dem weiterhin enthaltenen aliphatischen Alkohol besonders gut löslich ist. Eine weiterhin vorhandene Gruppe von Lösungsvermittlern ermöglicht eine bessere Verteilung des Gels auf der Haut. Die Zusammensetzung weist daher wenigstens einen ersten Lösungsvermittler auf, welcher die bessere Löslichkeit des NSAID ermöglicht, und wenigstens einen vom ersten Lösungsvermittler verschieden zweiten Lösungsvermittler auf, der eine verbesserte Verteilung auf der Haut ermöglicht. Dimethylisosorbid und/oder Glyceroldimethylketal sind dabei bevorzugt, um die verbesserte Löslichkeit des NSAID zu ermöglichen. Besonders bevorzugt ist Dimethylisosorbid. Isopropylmyristat sorgt insbesondere für eine bessere und damit gleichmäßigere Verteilung der Zusammensetzung auf der Haut.

Die erfindungsgemäße Zusammensetzung umfasst daher Lösungsvermittler Dimethylisosorbid und Isopropylmyristat.

Bevorzugt ist das Gewichtsverhältnis des ersten Lösungsvermittlers, welcher die bessere Löslichkeit des NSAID ermöglicht, zum zweiten Lösungsvermittler, eine verbesserte Verteilung auf der Haut ermöglicht, 10 : 1 bis 2 : 1, insbesondere 7 : 1 bis 2 : 1 und bevorzugt 5 : 1 bis 3 : 1.

Bevorzugt liegt der Anteil aller Lösungsvermittler in der erfindungsgemäßen Zusammensetzung in einem Bereich von 5 Gew.-% bis 35 Gew.-% oder von 5 Gew.-% bis 30 Gew.-%, besonders von 7 Gew.-% bis 25 Gew.-%, insbesondere von 7 Gew.-% bis 20 Gew.-%, bevorzugt von 8 Gew.-% bis 15 Gew.-%, jeweils bezogen auf das Gesamtgewicht der Zusammensetzung von 100 Gew.-%.

Es hat sich gezeigt, dass ein Gesamtanteil von 3 Gew.-% an allen Lösungsvermittlern notwendig ist, um das wenigstens eine NSAID in Lösung zu bringen. Dabei ist der Anteil an Lösungsvermittlern abhängig von der Menge des eingesetzten NSAID. Dabei sind 7 Gew.-% bis 25 Gew.-% ausreichend, um NSAID in einem Anteil von 3 Gew.-% bis 7 Gew.-% in Lösung zu bringen. Besonders bevorzugt beträgt der Anteil an Lösungsvermittlern 8 Gew.-% bis 15 Gew.-%. Die Gew.-% sind jeweils bezogen auf das Gesamtgewicht der Zusammensetzung von 100 Gew.-%.

Umfasst die erfindungsgemäße Zusammensetzung zwei verschiedene Lösungsvermittler, wie zuvor definiert, beträgt der Anteil des ersten Lösungsvermittlers vorzugsweise 2 Gew.-% bis 35 Gew.-%, insbesondere 5 Gew.-% bis 25 Gew.-%, bevorzugt 7 Gew.-% bis 14 Gew.-%. Der Anteil am zweiten Lösungsvermittler beträgt vorzugsweise 0,5 Gew.-% bis 10 Gew.-%, insbesondere von 1 Gew.-% bis 7 Gew.-%, besonders bevorzugt von 1,5 Gew.-% bis 5 Gew.-%, jeweils bezogen auf das Gesamtgewicht der Zusammensetzung von 100 Gew.-%.

Weiterhin weist die erfindungsgemäße Zusammensetzung wenigstens einen aliphatischen Alkohol in einem Anteil von 10 Gew.-% oder mehr auf. Der aliphatische Alkohol dient dabei als primäres Lösungsmittel für das NSAID, um dieses später zusammen mit Wasser in das Netzwerk des Strukturbildners einzubauen. Der wenigstens eine aliphatische Alkohol weist die allgemeine Formel CₙH₂ₙ₊₁OH auf, wobei n eine ganze natürliche Zahl von 2 bis 10 ist. Bevorzugt ist der aliphatische Alkohol ausgewählt aus Ethanol, Butanol, Propanol und/oder Hexanol. Dabei sind hier alle Isomere der genannten Alkohole umfasst. Besonders bevorzugt ist der aliphatische Alkohol Isopropanol. In diesem sind NSAID und insbesondere das bevorzugte Ibuprofen besonders gut löslich.

Der Anteil des wenigstens einen Alkohols liegt vorzugsweise in einem Bereich von 10 Gew.-% bis 40 Gew.-%, insbesondere von 12 Gew.-% bis 30 Gew.-%, besonders bevorzugt von 15 Gew.-% bis 25 Gew.-%, jeweils bezogen auf das Gesamtgewicht der Zusammensetzung von 100 Gew.-%. Auch hier ist der Anteil an Alkohol abhängig vom Anteil des NSAID, welches in der Zusammensetzung enthalten ist. Der Anteil an Alkohol liegt immer unterhalb des Anteils an Wasser, so dass Wasser das Hauptlösungsmittel darstellt.

Um das NSAID im Gel gleichmäßig zu verteilen, beträgt das Gewichtsverhältnis von NSAID zum Strukturbildner vorzugsweise 1 : 10 bis 10 : 1, insbesondere von 1 : 5 bis 5 : 1 und besonders bevorzugt von 1 : 5 bis 1 : 3. Hierdurch wird eine besonders gleichmäßige Wirkstoffverteilung erreicht und auch die Struktur des Gels ist angenehm für den Verbraucher.

In einer besonders bevorzugten Ausführungsform betrifft die vorliegende Erfindung eine Zusammensetzung, die aus
a. Ibuprofen als nicht-steroidalem Antiphlogistikum in einem Anteil von 1 Gew. -% bis 10 Gew.-%, insbesondere 2 Gew.-% bis 8 Gew.-%, besonders 3 Gew.-% bis 7 Gew.-%, bevorzugt 5 Gew.-%,
b. Poloxamer 407 als Strukturbildner in einem Anteil von 3 Gew.-% bis 40 Gew.-%, insbesondere von 5 Gew.-% bis 35 Gew.-% oder von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% oder von 8 Gew.-% bis 12 Gew.-%,
c. Dimethylisosorbid (DMIS) als erstem Lösungsvermittler und Isopropylmyristat (IPM) als zweitem Lösungsvermittler, wobei die Gesamtmenge an Lösungsvermittler von 3 Gew.-% bis 40 Gew.-%, insbesondere von 5 Gew.-% bis 35 Gew.-% oder von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% beträgt,
d. Isopropanol als alkoholisches Lösungsmittel in einem Anteil von wenigstens 10 Gew.-%, insbesondere von 10 Gew.-% bis 40 Gew.-%, besonders von 12 Gew.-% bis 30 Gew.-%, bevorzugt von 15 Gew.-% bis 25 Gew.-%, und
e. Wasser in einem Anteil von 40 Gew.-% oder mehr, besteht.

Soweit in der vorliegenden Anmeldung Gew.-% von Zusammensetzungen genannt sind, beziehen sie sich diese jeweils auf das Gesamtgewicht der Zusammensetzung, welches 100 Gew.-% beträgt.

Die Zusammensetzung weist Dimethylisosorbid und Isopropylmyristat als ersten und zweiten Lösungsvermittler auf. Bevorzugt ist das Gewichtsverhältnis von Dimethylisosorbid zu Isopropylmyristat 10 : 1 bis 2 : 1, insbesondere 7 : 1 bis 2 : 1 und bevorzugt 5 : 1 bis 3 : 1. Der Anteil an Dimethylisosorbid beträgt vorzugsweise 2 Gew.-% bis 35 Gew.-%, insbesondere 5 Gew.-% bis 25 Gew.-%, bevorzugt 7 Gew.-% bis 14 Gew.-%. Der Anteil an Isopropylmyristat beträgt vorzugsweise 0,5 Gew.-% bis 10 Gew.-%, insbesondere von 1 Gew.-% bis 7 Gew.-%, besonders bevorzugt von 1,5 Gew.-% bis 5 Gew.-%, jeweils bezogen auf das Gesamtgewicht der Zusammensetzung von 100 Gew.-%.

Die erfindungsgemäße Zusammensetzung kann nach an sich bekannten Herstellungsverfahren hergestellt werden. Die Formulierung hat den besonderen Vorteil, dass sich die Struktur beim Auftragen auf die Haut nicht verändert. Dabei bleibt die effektive Permeation des Wirkstoffes in die Haut verglichen mit im Stand der Technik bekannten Zusammensetzungen wenigstens erhalten. Weiterhin ist die erfindungsgemäße Formulierung lagerfähig, so dass auch eine länger andauernde Anwendung durch den Verbraucher ermöglicht wird.

Alle Angaben in % beziehen sich, soweit nicht anders angegeben, auf Gew.-%; Gew.-% beziehen sich dabei auf ein Gesamtgewicht der Zusammensetzung von 100 Gew.-%. Sind Verhältnisse angegeben, so ist hierunter das Gewichtsverhältnis der genannten Komponenten zu verstehen. Soweit der Begriff "umfassen" in der vorliegenden Anmeldung genannt ist, ist die Bedeutung von "bestehen aus" mit umfasst. Die zuvor genannten einzelnen Merkmale können erfindungsgemäß beliebig miteinander kombiniert werden.

In der nachfolgenden Aufzählung werden bevorzugte Ausgestaltungen der vorliegenden Erfindung kurz ausgeführt.

Im nachfolgenden Ausführungsbeispiel wird die vorliegende Erfindung in nicht limitierender Weise genauer erläutert.

### Ausführungsbeispiel:

Es wurde ein Gel mit der folgenden Zusammensetzung hergestellt.

| | |
|---|---|
| Ibuprofen | 5,0 Gew.-% |
| Isopropanol | 20,5 Gew.-% |
| Poloxamer 407 | 10,4 Gew.-% |
| Dimethylisosorbid | 7,5 Gew.-% |
| Isopropyl myristat | 2,0 Gew.-% |
| gereinigtes Wasser | 54,6 Gew.-% |

Das erhaltene Gel konnte angenehm auf die Haut aufgebracht werden, ohne dass dies zu Verklumpungen des Gels oder der Ausbildung eines unangenehm wirkenden Films auf der Haut führte. Das Gel war transparent, behielt bei Raumtemperatur die äußere Form und lies sich gut auf der Haut auftragen.

## Patentansprüche

1. Wässrige Zusammensetzung in Form eines Gels für die transdermale Aufnahme wenigstens eines nicht-steroidalen Antiphlogistikums (NSAID) bestehend aus:
a. wenigstens einem nicht-steroidales Antiphlogistikum in einem Anteil von 1 Gew.-% bis 10 Gew.-%,
b. wenigstens einem Verdickungsmittel, welches in der Lage ist, ein dreidimensionales Netzwerk eines Gels auszubilden, in einem Anteil von 3 Gew.-% bis 40 Gew.-%,
c. Dimethylisosorbid als ersten Lösungsvermittler zur Verbesserung der Löslichkeit des wenigstens einen NSAID in einem aliphatischen Alkohol in einem Anteil von 2 Gew.-% bis 35 Gew.-%,
d. Isopropylmyristat als zweiten Lösungsvermittler zur Verbesserung der Verreibbarkeit der Zusammensetzung auf der Haut, in einem Anteil von 0,5 Gew.-% bis 10 Gew.-%,
e. wenigstens einen aliphatischen Alkohol der allgemeinen Formel CₙH₂ₙ₊₁OH, wobei n eine ganze natürliche Zahl von 2 bis 10 ist, einem Anteil von 10 Gew.-% oder mehr und
f. Wasser in einem Anteil von 40 Gew.-% oder mehr,
wobei die Gesamtmenge an Lösungsvermittler 3 Gew.-% bis 40 Gew.-% beträgt und das Gewichtsverhältnis von erstem Lösungsvermittler zu zweitem Lösungsvermittler 10:1 bis 2:1 beträgt, und
das Gesamtgewicht der Zusammensetzung 100 Gew.-% beträgt.

2. Zusammensetzung nach Anspruch 1, **dadurch gekennzeichnet, dass** sie drei oder mehr Lösungsvermittler aufweist.

3. Zusammensetzung nach Anspruch 1 oder 2, **dadurch gekennzeichnet, dass** sie ein oder mehrere, insbesondere ein nicht-steroidales Antiphlogistikum, ausgewählt aus Ibuprofen, Flurbiprofen, Naproxen, Ketoprofen und Tiaprofensäure, insbesondere Ibuprofen, aufweist.

4. Zusammensetzung nach einem der Ansprüche 1 bis 3, **dadurch gekennzeichnet, dass** sie das wenigstens eine NSAID in einem Anteil von 2 Gew.-% bis 8 Gew.-%, insbesondere von 3 Gew.-% bis 7 Gew.-%, bevorzugt 5 Gew.-%, bezogen auf das Gesamtgewicht der Zusammensetzung von 100 Gew.-%, aufweist.

5. Zusammensetzung nach einem der Ansprüche 1 bis 4, **dadurch gekennzeichnet, dass** das wenigstens eine Verdickungsmittel ein amphiphiles Blockcopolymer aus Ethylenoxid und Propylenoxid ist.

6. Zusammensetzung nach einem der Ansprüche 1 bis 5, **dadurch gekennzeichnet, dass** sie das wenigstens eine Verdickungsmittel in einem Anteil von 5 Gew.-% bis 35 Gew.-%, insbesondere von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% oder von 8 Gew.-% bis 12 Gew.-%, bezogen auf das Gesamtgewicht der Zusammensetzung von 100 Gew.-%, aufweist.

7. Zusammensetzung nach einem der Ansprüche 1 bis 6, **dadurch gekennzeichnet, dass** die Gesamtmenge an Lösungsvermittler in einem Bereich von 5 Gew.-% bis 35 Gew.-% oder von 5 Gew.-% bis 30 Gew.-%, besonders von 7 Gew.-% bis 25 Gew.-%, insbesondere von 7 Gew.-% bis 20 Gew.-%, bevorzugt von 8 Gew.-% bis 15 Gew.-%, bezogen auf das Gesamtgewicht der Zusammensetzung von 100 Gew.-%, liegt.

8. Zusammensetzung nach einem der Ansprüche 1 bis 7, **dadurch gekennzeichnet, dass** der wenigstens eine aliphatische Alkohol ausgewählt ist aus Ethanol, Butanol, Propanol und/oder Hexanol, insbesondere Isopropanol.

9. Zusammensetzung nach einem der Ansprüche 1 bis 8, **dadurch gekennzeichnet, dass** sie den wenigstens einen Alkohol in einem Anteil von 10 Gew.-% bis 40 Gew.-%, insbesondere von 12 Gew.-% bis 30 Gew.-%, bevorzugt von 15 Gew.-% bis 25 Gew.-%, bezogen auf das Gesamtgewicht der Zusammensetzung von 100 Gew.-%, aufweist.

10. Zusammensetzung nach einem der Ansprüche 1 bis 9, **dadurch gekennzeichnet, dass** das Gewichtsverhältnis von NSAID zu Verdickungsmittel im Bereich von 1:10 bis 10:1, insbesondere von 1:5 bis 5:1 und besonders von 1:5 bis 1:3 liegt.

11. Zusammensetzung nach einem der Ansprüche 1 bis 10, **dadurch gekennzeichnet, dass** sie aus
a. Ibuprofen als nicht-steroidalem Antiphlogistikum in einem Anteil von 1 Gew. -% bis 10 Gew.-%, insbesondere 2 Gew.-% bis 8 Gew.-%, besonders 3 Gew.-% bis 7 Gew.-%, bevorzugt 5 Gew.-%,
b. Poloxamer 407 als Verdickungsmittel in einem Anteil von 3 Gew.-% bis 40 Gew.-%, insbesondere von 5 Gew.-% bis 35 Gew.-% oder von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% oder von 8 Gew.-% bis 12 Gew.-%, und
c. Dimethylisosorbid (DMIS) als ersten Lösungsvermittler und
d. Isopropylmyristat (IPM) als zweiten Lösungsvermittler,
e. Isopropanol als alkoholisches Lösungsmittel in einem Anteil von wenigstens 10 Gew.-%, insbesondere von 10 Gew.-% bis 40 Gew.-%, besonders von 12 Gew.-% bis 30 Gew.-%, bevorzugt von 15 Gew.-% bis 25 Gew.-%, und
f. Wasser in einem Anteil von 40 Gew.-% oder mehr,
besteht, wobei die Gesamtmenge an Lösungsvermittler von 3 Gew.-% bis 40 Gew.-%, insbesondere von 5 Gew.-% bis 35 Gew.-% oder von 5 Gew.-% bis 30 Gew.-%, besonders von 7 Gew.-% bis 25 Gew.-% oder von 7 Gew.-% bis 20 Gew.-%, bevorzugt von 8 Gew.-% bis 15 Gew.-% beträgt, und
das und Gesamtgewicht der Zusammensetzung 100 Gew.-% beträgt.

12. Zusammensetzung nach Anspruch 11, **dadurch gekennzeichnet, dass** das Gewichtsverhältnis von Dimethylisosorbid (DMIS) zu Isopropylmyristat (IPM) 10:1 bis 2:1, insbesondere 7:1 bis 2:1 und bevorzugt 5:1 bis 3:1 beträgt.

13. Zusammensetzung nach Anspruch 11 oder 12, **dadurch gekennzeichnet, dass** der Anteil an Dimethylisosorbid (DMIS) von 2 Gew.-% bis 35 Gew.-%, insbesondere von 5 bis 25 Gew.-%, bevorzugt von 7 bis 14 Gew.-% beträgt.

14. Zusammensetzung nach einem der Ansprühe 11 bis 13, **dadurch gekennzeichnet, dass** der Anteil an Isopropylmyristat (IPM) 0,5 Gew.-% bis 10 Gew.-%, insbesondere von 1 Gew.-% bis 7 Gew.-%, bevorzugt von 1,5 Gew.-% bis 5 Gew.-% beträgt.

15. Zusammensetzung nach einem der Ansprüche 1 bis 14 zur Anwendung zur Schmerzlinderung.

## Claims

1. An aqueous composition in the form of a gel for the transdermal uptake of at least one non-steroidal anti-inflammatory drug (NSAID), consisting of:
a. at least one non-steroidal anti-inflammatory drug in a proportion of 1% by weight to 10% by weight;
b. at least one thickening agent that is capable of forming a three-dimensional network of a gel, in a proportion of 3% by weight to 40% by weight;
c. dimethylisosorbide as a first solubilizer for improving the solubility of said at least one NSAID in an aliphatic alcohol, in a proportion of 2% by weight to 35% by weight;
d. isopropyl myristate as a second solubilizer for improving the capability of the composition of being rubbed on the skin, in a proportion of 0.5% by weight to 10% by weight;
e. at least one aliphatic alcohol of general formula CₙH₂ₙ₊₁OH, in which n is a whole natural number of from 2 to 10, in a proportion of 10% by weight or more; and
f. water in a proportion of 40% by weight or more;
wherein the total amount of solubilizer is from 3% by weight to 40% by weight, and the weight ratio of said first solubilizer to said second solubilizer is from 10:1 to 2:1; and
the total weight of the composition is 100% by weight.

2. The composition according to claim 1, **characterized by** containing three or more solubilizers.

3. The composition according to claim 1 or 2, **characterized by** containing one or more, especially one, non-steroidal anti-inflammatory drug selected from ibuprofen, flurbiprufen, naproxen, ketoprofen, and tiaprofenic acid, especially ibuprofen.

4. The composition according to any of claims 1 to 3, **characterized by** containing said at least one NSAID in a proportion of 2% by weight to 8% by weight, especially 3% by weight to 7% by weight, preferably 5% by weight, based on the total weight of the composition of 100% by weight.

5. The composition according to any of claims 1 to 4, **characterized in that** said at least one thickening agent is an amphiphilic block copolymer of ethylene oxide and propylene oxide.

6. The composition according to any of claims 1 to 5, **characterized by** containing said at least one thickening agent in a proportion of 5% by weight to 35% by weight, especially 5% by weight to 30% by weight, especially 7% by weight to 25% by weight, or 7% by weight to 20% by weight, preferably 8% by weight to 15% by weight, or 8% by weight to 12% by weight, based on the total weight of the composition of 100% by weight.

7. The composition according to any of claims 1 to 6, **characterized in that** the total amount of solubilizers is within a range of from 5% by weight to 35% by weight, or 5% by weight to 30% by weight, especially 7% by weight to 25% by weight, especially 7% by weight to 20% by weight, preferably 8% by weight to 15% by weight, based on the total weight of the composition of 100% by weight.

8. The composition according to any of claims 1 to 7, **characterized in that** said at least one aliphatic alcohol is selected from ethanol, butanol, propanol, and/or hexanol, especially isopropanol.

9. The composition according to any of claims 1 to 5, **characterized by** containing said at least one alcohol in a proportion of 10% by weight to 40% by weight, especially 12% by weight to 30% by weight, preferably 15% by weight to 25% by weight, based on the total weight of the composition of 100% by weight.

10. The composition according to any of claims 1 to 9, **characterized in that** the weight ratio of NSAID to thickening agent is within a range of from 1:10 to 10:1, especially from 1:5 to 5:1, and especially from 1:5 to 1:3.

11. The composition according to any of claims 1 to 10, **characterized by** consisting of
a. ibuprofen as a non-steroidal anti-inflammatory drug in a proportion of 1% by weight to 10% by weight, especially 2% by weight to 8% by weight, especially 3% by weight to 7% by weight, preferably 5% by weight;
b. poloxamer 407 as a thickening agent in a proportion of 3% by weight to 40% by weight, especially 5% by weight to 35% by weight, especially 7% by weight to 25% by weight, or 7% by weight to 20% by weight, preferably 8% by weight to 15% by weight, or 8% by weight to 12% by weight; and
c. dimethylisosorbide (DMIS) as a first solubilizer; and
d. isopropyl myristate (IPM) as a second solubilizer;
e. isopropanol as an alcoholic solvent in a proportion of at least 10% by weight, especially 10% by weight to 40% by weight, especially 12% by weight to 30% by weight, preferably 15% by weight to 25% by weight; and
f. water in a proportion of 40% by weight or more;
wherein the total amount of solubilizer is from 3% by weight to 40% by weight, especially 5% by weight to 35% by weight, or 5% by weight to 30% by weight, especially 7% by weight to 35% by weight, or 7% by weight to 20% by weight, preferably 8% by weight to 15% by weight; and
the total weight of the composition is 100% by weight.

12. The composition according to claim 11, **characterized in that** the weight ratio of dimethylisosorbide (DMIS) to isopropyl myristate (IPM) is from 10:1 to 2:1, especially from 7:1 to 2:1, and preferably from 5:1 to 3:1.

13. The composition according to claim 11 or 12, **characterized in that** the proportion of dimethylisosorbide (DMIS) is from 2% by weight to 35% by weight, especially 5% to 25% by weight, preferably 7% to 14% by weight.

14. The composition according to any of claims 11 to 13, **characterized in that** the proportion of isopropyl myristate (IPM) is from 0.5% by weight to 10% by weight, especially 1% to 7% by weight, preferably 1.5% to 5% by weight.

15. The composition according to any of claims 1 to 14 to be applied for relieving pain.

## Revendications

1. Composition aqueuse sous forme d'un gel pour l'absorption par voie trans-dermique d'au moins un médicament anti-inflammatoire non-stéroïdien (NSAID) consistant en :
a. au moins un médicament anti-inflammatoire non-stéroïdien à raison de 1 % en poids à 10 % en poids,
b. au moins un épaississant qui peut former un réseau tridimensionnel d'un gel, à raison de 3 % en poids à 40 % en poids,
c. diméthylisosorbide comme premier agent solubilisant pour améliorer la solubilité dudit au moins un NSAID dans un alcool aliphatique, à raison de 2 % en poids à 35 % en poids,
d. myristate d'isopropyle comme deuxième agent solubilisant pour améliorer la frottabilité de la composition sur la peau, à raison de 0,5 % en poids à 10 % en poids,
e. au moins un alcool aliphatique répondant à la formule générale CₙH₂ₙ₊₁OH, où n est un nombre naturel entier de 2 à 10, à raison de 10 % en poids ou plus, et
f. de l'eau à raison de 40 % en poids ou plus,
dans laquelle la quantité totale d'agents solubilisants est de 3 % en poids à 40 % en poids, et le rapport pondéral du premier agent solubilisant au deuxième agent solubilisant est de 10 : 1 à 2 : 1, et
le poids total de la composition est 100 % en poids.

2. Composition selon la revendication 1, **caractérisée en ce qu'**elle comprend trois ou plusieurs agents solubilisants.

3. Composition selon la revendication 1 ou 2, **caractérisée en ce qu'**elle comprend un ou plusieurs, notamment un, médicament anti-inflammatoire non-stéroïdien choisi parmi ibuprofène, flurbiprofène, naproxène, kétoprofène, et acide tiaprofénique, notamment ibuprofène.

4. Composition selon l'une quelconque des revendications 1 à 3, **caractérisée en ce qu'**elle comprend ledit au moins un NSAID à raison de 2 % en poids à 8 % en poids, notamment de 3 % en poids à 7 % en poids, de préférence 5 % en poids, par rapport au poids total de la composition de 100 % en poids.

5. Composition selon l'une quelconque des revendications 1 à 4, **caractérisée en ce que** ledit au moins un épaississant est un copolymère séquencé amphiphile d'oxyde d'éthylène et d'oxyde de propylène.

6. Composition selon l'une quelconque des revendications 1 à 5, **caractérisée en ce qu'**elle comprend ledit au moins un épaississant à raison de 5 % en poids à 35 % en poids, notamment de 5 % en poids à 30 % en poids, notamment de 7 % en poids à 25 % en poids, ou de 7 % en poids à 20 % en poids, de préférence de 8 % en poids à 15 % en poids, ou de 8 % en poids à 12 % en poids, par rapport au poids total de la composition de 100 % en poids.

7. Composition selon l'une quelconque des revendications 1 à 6, **caractérisée en ce que** la quantité totale d'agents solubilisants est comprise entre 5 % en poids et 35 % en poids, ou entre 5 % en poids et 30 % en poids, notamment entre 7 % en poids et 25 % en poids, notamment entre 7 % en poids et 20 % en poids, de préférence entre 8 % en poids et 15 % en poids, par rapport au poids total de la composition de 100 % en poids.

8. Composition selon l'une quelconque des revendications 1 à 7, **caractérisée en ce que** ledit au moins un alcool aliphatique est choisi parmi éthanol, butanol, propanol, et/ou hexanol, notamment isopropanol.

9. Composition selon l'une quelconque des revendications 1 à 7, **caractérisée en ce qu'**elle comprend ledit au moins un alcool à raison de 10 % en poids à 40 % en poids, notamment de 12 % en poids à 30 % en poids, de préférence de 15 % en poids à 25 % en poids, par rapport au poids total de la composition de 100 % en poids.

10. Composition selon l'une quelconque des revendications 1 à 9, **caractérisée en ce que** le rapport pondéral de NSAID à l'épaississant est compris entre 1 : 10 et 10 : 1, notamment entre 1 : 5 et 5 : 1, et notamment entre 1 : 5 et 1 : 3.

11. Composition selon l'une quelconque des revendications 1 à 10, **caractérisée en ce qu'**elle consiste en
a. ibuprofène comme médicament anti-inflammatoire non-stéroïdien à raison de 1 % en poids à 10 % en poids, notamment de 2 % en poids à 8 % en poids, notamment de 3 % en poids à 7 % en poids, de préférence 5 % en poids,
b. poloxamère 407 comme épaississant à raison de 3 % en poids à 40 % en poids, notamment de 5 % en poids à 35 % en poids, ou de 5 % en poids à 30 % en poids, notamment de 7 % en poids à 25 % en poids, ou de 7 % en poids à 20 % en poids, de préférence de 8 % en poids à 15 % en poids, ou de 8 % en poids à 12 % en poids, et
c. diméthylisosorbide (DMIS) comme premier agent solubilisant, et
d. myristate d'isopropyle (IPM) comme deuxième agent solubilisant,
e. isopropanol comme solvant alcoolique à raison d'au moins 10 % en poids, notamment de 10 % en poids à 40 % en poids, notamment de 12 % en poids à 30 % en poids, de préférence de 15 % en poids à 25 % en poids, et
f. de l'eau à raison de 40 % en poids ou plus,
dans laquelle la quantité totale d'agents solubilisants est de 3 % en poids à 40 % en poids, notamment de 5 % en poids à 35 % en poids, ou de 5 % en poids à 30 % en poids, notamment de 7 % en poids à 25 % en poids, ou de 7 % en poids à 20 % en poids, de préférence de 8 % en poids à 15 % en poids, et
le poids total de la composition est 100 % en poids.

12. Composition selon la revendication 11, **caractérisée en ce que** le rapport pondéral de diméthylisosorbide (DMIS) à myristate d'isopropyle (IPM) est de 10 : 1 à 2 : 1, notamment 7 : 1 à 2 : 1, et de préférence 5 : 1 à 3 : 1.

13. Composition selon la revendication 11 ou 12, **caractérisée en ce que** la fraction de diméthylisosorbide (DMIS) est de 2 % en poids à 35 % en poids, notamment de 5 à 25 % en poids, de préférence de 7 à 14 % en poids.

14. Composition selon l'une quelconque des revendications 11 à 13, **caractérisée en ce que** la fraction de myristate d'isopropyle (IPM) est de 0,5 % en poids à 10 % en poids, notamment de 1 % en poids à 7 % en poids, de préférence de 1,5 % en poids à 5 % en poids.

15. Composition selon l'une quelconque des revendications 1 à 14 à appliquer pour le soulagement de la douleur.
